# EUROPEAN PATENT SPECIFICATION

(11) **EP 0 946 874 B1**
(45) Date of publication and mention of the grant of the patent: **05.06.2002**
(21) Application number: 97953172.0
(22) Date of filing: 18.12.1997
(51) Int. Cl.: G01N 33/569, G01N 33/543

(54) **HELICOBACTER PYLORI DIAGNOSTICS**
HELICOBACTER PYLORI DIAGNOSTIKA
DIAGNOSTICS DE L'HELICOBACTER PYLORI

(30) Priority: 19.12.1996 US 33707 P
(43) Date of publication of application: 06.10.1999
(73) Proprietor: CHIRON CORPORATION, Emeryville, California 94608 (US)
(72) Inventor: QUAN, Stella, Emeryville, CA 94608 (US); VALENZUELA, Pablo, Berkeley, CA 94705 (US); POLITO, Alan, Emeryville, CA 94608 (US)
(74) Representative: Hallybone, Huw George
(86) International application number: US9722798
(87) International publication number: WO9827432

(56) References cited:
- WO-A-93/18150
- CHEMICAL ABSTRACTS, vol. 126, no. 1, 7 July 1997 Columbus, Ohio, US; abstract no. 747006, XP002063245 & S. CENSINI ET AL.: "CAG, a pathogenicity island of Helicobacter pylori, encodes types I-specific and disease-associated virulence factors " PROCEEDINGS OF THE NATIONAL ACADEMY OF SCIENCES USA, vol. 93, no. 25, 1 December 1996, WASHINGTON DC USA, pages 14648-14653,

## Description

### Background of the Invention

### Technical Field

The present invention pertains generally to bacterial diagnostic techniques. In particular, the invention relates to methods for accurately detecting *Helicobacter pylori* infection in a biological sample and for monitoring the course of antibiotic treatment in a patient with an *H. pylori* infection.

### Background of the Invention

*Helicobacter pylori,* originally named *Campylobacter pylori,* is a curved, microaerophilic, gram-negative bacterium that exhibits high urease and catalase activity. Recent studies suggest that *H. pylori* infection may be either a cause of, or a cofactor in, type B gastritis, peptic ulcers, and gastric tumors. See, e.g., Blaser, *Gastroenterology* (1987) 93:371-383; Dooley et al., *New Eng. J. Med.* (1989) 321:1562-1566; Personnet et al., *New Eng. J. Med.* (1991) 325:1127-1131. In this regard, *H. pylori* colonizes the human gastric mucosa and causes an infection that can persist for decades. Many people with this condition are asymptomatic but are nonetheless at a considerable risk of developing peptic ulcers and/or gastric adenocarcinomas. For a review of *H. pylori* and its role in gastric disease, see, Telford et al., *Trends in Biotech.* (1994) 12:420-426 and Blaser, M.J., *Scientific* American (February 1996):104-107.

*H. pylori* bacteria are divided into two groups, Type I and Type II, based on the presence or absence of specific proteins. In this regard, *H*. *pylori* produces several factors that function to establish and maintain infection. For example, both Type I and Type II bacteria include flagella that aid in mobility in the viscous mucus layer of the stomach. Both types of bacteria also produce ureases, presumably to neutralize the acid environment of the stomach. Additionally, the two types of bacteria produce a number of adhesins for tissue-specific colonization. On the other hand, only *H*. *pylori* Type I strains produce a potent cytotoxin, known as VacA or CT, as well as a surface-exposed immunodominant antigen which is associated with cytotoxin expression, known as CagA, CAI antigen or tagA. For descriptions of VacA and CagA, see, e.g., International Publication No. WO 93/18150, published 16 September 1993.

Patients with duodenal ulcers have been shown to produce antibodies to VacA and CagA and antibody titers appear to correlate with the severity of the disease. For example, in one study, more than 95% of patients with duodenal ulcer or duodenitis, and more than 70% of patients suffering from gastric ulcer, were found to be CagA seropositive. Telford et al., *Trends in Biotech.* (1994) 12:420-426. Furthermore, a correlation has been shown between CagA serum response and gastric adenocarcinoma. Telford et al., *supra.* Additionally, only cytotoxic strains are able to induce gastric lesions in a laboratory animal model. See, e.g., Telford et al., *J. Exp. Med.* (1994) 179:1653-1658. Thus, it is believed that only individuals infected with *H*. *pylori* Type I strains develop severe disease.

Several assays have been developed for the diagnosis of *H. pylori* infection. These assays,. unfortunately, suffer from several drawbacks. For example, bacterial culture assays have been described for the detection of *H. pylori.* U.S. Patent No. 5,498,528 describes such a method for detecting *H. pylori* in saliva. The assay requires incubating the test sample with a culture medium that supports the selective growth of *H*. *pylori.* The presence of the bacterium is detected by the activity of the enzyme urease which, as described above, is produced by *H*. *pylori.* Urease catalyzes the conversion of urea to ammonium causing an increase in the pH of the culture medium. The pH change can be detected by a color change to the medium due to the presence of a pH sensitive indicator. However, the assay is time consuming since the bacteria require a number of days for growth. The assay is also inconvenient and bacterial samples may degrade or become contaminated during transport to the laboratory.

Antibody detection tests provide an alternative to bacterial culture. In this regard, subjects colonized with *H. pylori* mount a humoral immune response and produce antibodies to the bacterium that can be used as a basis for diagnosis. IgA antibodies are found in gastric fluid while IgG antibodies are found in the circulation. However, such tests can suffer from a lack of specificity since *H. pylori* appears to be antigenically cross-reactive with *Campylobacter* jejuni and *C. coli.*

U.S. Patent No. 4,882,271 describes an *H. pylori* assay that utilizes high molecular weight cell-associated proteins, on the order of 300 kDa to 700 kDa, having urease activity, in an enzyme-linked immunosorbent assay (ELISA), in an attempt to circumvent the problems with cross-reactivity.

International Publication No. WO 96/12965, published 2 May 1996, describes an immunoblot assay where a serological sample is reacted with two antigen components having molecular weights of 19.5 kDa, 26.5 kDa or 30 kDa, or alternatively, any one antigen component corresponding to a molecular weight of 35 kDa, 89 kDa, 116 kDa or 180 kDa. It is postulated by the inventors that the 19.5 kDa protein is a ferritin-like protein, the 26.5 and 30 kDa proteins are ureases, the 89 kDa protein is VacA, and that the 116 kDa protein is CagA. The 35 kDa and 180 kDa were uncharacterized.

Finally, European Patent Publication 329,570, published 23 August 1989, describes immunoassays for *H. pylori* infection using pooled suspensions of sonicates of several *H. pylori* strains, as well as immunoassays using purified *H. pylori* flagellae.

Although faster and more sensitive than bacterial culture, antibody detection tests, such as those described above, can give false positive and negative results and generally do not distinguish between *H. pylori* Type I and Type II infection. Thus, an additional test must be conducted to determine whether the infection is due to *H. pylori* Type I or Type II.

Accordingly, the wide spread availability of an accurate and efficient assay for *H. pylori* infection that readily distinguishes between Type I and Type II infection, would be important for the diagnosis of infection in both symptomatic and asymptomatic individuals.

### Summary of the Invention

The present invention provides a simple, extremely accurate and efficient method for diagnosing *H. pylori* infection, as well as for distinguishing between *H*. *pylori* Type I and *H. pylori* Type II infections. Thus, the method provides a technique for screening for individuals with *H*. *pylori* Type I infection. If Type I infection is detected, the individual can be given antibiotics to treat or prevent type B gastritis, peptic ulcers, and gastric tumors. The method is also useful for monitoring the course of treatment in a patient with an *H. pylori* infection. The assay method utilizes both type-common antigens, as well as particular type-specific antigens from the bacterium.

Accordingly, in one embodiment, the subject invention is directed to a method of detecting *H. pylori* infection comprising:
(a) providing a biological sample;
(b) reacting the biological sample with one or more *H. pylori* type-common antigens and reacting the biological sample with one or more purified type-specific *H*. *pylori* Type I antigens, under conditions which allow *H. pylori* antibodies, when present in the biological sample, to bind with the *H*. *pylori* type-common antigens and/or the type-specific antigens,
thereby detecting the presence or absence of *H. pylori* infection.

In other embodiments, the invention is directed to a method for distinguishing between *H*. *pylori* Type I and *H. pylori* Type II infection in a biological sample, or a method of monitoring a subject undergoing therapy for an *Helicobacter pylori* infection, the methods comprising:
(a) immobilizing one or more *H*. *pylori* type-common antigens, e.g., an *H. pylori* lysate and/or *H. pylori* urease, and immobilizing one or more purified type-specific *H. pylori* Type I antigens, e.g., *H. pylori* VacA and/or *H. pylori* CagA, on a nitrocellulose strip;
(b) contacting the nitrocellulose strip from step (a) with the biological sample under conditions which allow anti-*H*. *pylori* Type I and anti-*H*. *pylori* Type II antibodies, when present in the biological sample, to bind with *H. pylori* type-common antigens present in the lysate and/or the type-specific *H. pylori* Type I antigens;
(c) removing unbound antibodies;
(d) providing a detectably labeled anti-human immunoglobulin antibody; and
(e) detecting the presence or absence of bound anti-human immunoglobulin antibodies in the biological sample,
thereby detecting the presence or absence of *H. pylori* Type I or Type II infection.

In particularly preferred embodiments, the biological sample is a human serum sample.

In yet further embodiments, the invention is directed to membrane supports comprising one or more *H. pylori* type-common antigens and one or more purified type-specific *H. pylori* Type I antigens, discretely immobilized thereon.

In another embodiment, the invention is directed to a nitrocellulose support comprising:
(a) an *H. pylori* Type I VacA polypeptide;
(b) an *H*. *pylori* Type I CagA polypeptide;
(c) an *H*. *pylori* urease; and
(d) a human IgG,
wherein the *H*. *pylori* polypeptides and urease, and the human IgG, are immobilized as discrete bands on said nitrocellulose support.

In a further embodiment, the invention is directed to a nitrocellulose support comprising:
(a) an *H. pylori* Type I VacA polypeptide;
(b) an *H. pylori* Type I CagA polypeptide;
(c) an *H*. *pylori* lysate; and
(d) a human IgG,
wherein the *H*. *pylori* polypeptides and lysate, and the human IgG, are immobilized as discrete, bands on said nitrocellulose support.

In other embodiments, the invention is directed to immunodiagnostic test kits for detecting *H. pylori* infection. The kits comprise (a) one or more *H*. *pylori* type-common antigens; (b) one or more purified type-specific *H. pylori* Type I antigens; and (c) instructions for conducting the immunodiagnostic test.

In still further embodiments, the invention is directed to an immunodiagnostic test kit for distinguishing between *H. pylori* Type I and *H. pylori* Type II infection in a biological sample, or for monitoring a subject undergoing therapy for an *Helicobacter pylori* infection. The test kit comprises (a) one or more *H. pylori* type-common antigens immobilized on a nitrocellulose strip, e.g., an *H. pylori* lysate and/or *H*. *pylori* urease; (b) one or more purified type-specific *H. pylori* Type I antigens, e.g., *H. pylori* VacA and/or *H. pylori* CagA, immobilized on a nitrocellulose strip; and (c) instructions for conducting the immunodiagnostic test.

These and other embodiments of the present invention will readily occur to those of ordinary skill in the art in view of the disclosure herein.

### Brief Description of the Figures

Figure 1 depicts a representative test strip for use in a strip immunoblot assay (SIA). Human IgG is used as an internal control at two different levels (Level I, low control; and Level II, high control). CagA and VacA are used as the type-specific *H. pylori* Type I antigens and HP CE denotes the *H*. *pylori* lysate which contains type-common antigens.

Figure 2 shows another representative test strip for use in an SIA. As above, human IgG is used as an internal control at two different levels (Level I, low control; and Level II, high control). CagA and VacA are used as the type-specific *H*. *pylori* Type I antigens and are also used at two different levels to enhance the sensitivity of the assay as well as to monitor response to treatment. Urease is used as the type-common antigen.

Figures 3A-3B (SEQ ID NOS:____) show the nucleotide sequence and corresponding amino acid sequence for the *H*. *pylori* VacA antigen used in the SIAs described in the examples.

Figure 4 (SEQ ID NO:____) shows the nucleotide sequence and corresponding amino acid sequence for the *H*. *pylori* CagA antigen used in the SIAs described in the examples.

### Detailed Description of the Invention

The practice of the present invention will employ, unless otherwise indicated, conventional methods of immunology, microbiology, molecular biology and recombinant DNA techniques within the skill of the art. Such techniques are explained fully in the literature. See, e.g., Sambrook, et al., *Molecular Cloning: A Laboratory Manual* (2nd Edition, 1989); *DNA Cloning: A Practical Approach,* vol. I & II (D. Glover, ed.); *Methods In Enzymology* (S. Colowick and N. Kaplan eds., Academic Press, Inc.); and *Handbook of Experimental Immunology,* Vols. I-IV (D.M. Weir and C.C. Blackwell eds., Blackwell Scientific Publications).

As used in this specification and the appended claims, the singular forms "a," "an" and "the" include plural references unless the content clearly dictates otherwise. Additionally, standard abbreviations for nucleotides and amino acids are used in this specification.

### I. Definitions

In describing the present invention, the following terms will be employed, and are intended to be defined as indicated below.

By "an *H*. *pylori* lysate" is meant an extract or lysate derived from an *H*. *pylori* Type I or Type II whole bacterium which includes one or more *H. pylori* polypeptides, as defined below, that reacts with antibodies generated against both of *H. pylori* Type I and *H. pylori* Type II. Such polypeptides are termed "type-common" antigens herein. Thus the term "lysate" as used herein refers to crude extracts that contain several *H. pylori* antigens, so long as at least one of the antigens present in the lysate is a type-common antigen. The lysate can be augmented with additional purified type-common and/or type-specific antigens. The term also denotes relatively purified compositions derived from such crude lysates which include only one or few such type-common antigens. Such lysates are prepared using techniques well known in the art, described further below.

Representative antigens that may be present in such lysates, either alone or in combination, include one or more type-common epitopes derived from the *H. pylori* adhesins such as, but not limited to, a 20 kDa N-acetyl-neuraminillactose-binding fibrillar haemagglutinin (HpaA), a 63 kDa protein that binds phosphatidylethanolamine and gangliotetraosyl ceramide, and a conserved fimbrial pilus-like structure. See, e.g., Telford et al., *Trends in Biotech.* (1994) 12:420-426 for a description of these antigens. Other type-common antigens that may be present in the lysate include one or more type-common epitopes derived from any of the various flagellins such as the major flagellin, FlaA and the minor flagellin, FlaB. In this regard, the flagella of *H. pylori* are composed of FlaA and FlaB, each with a molecular weight of approximately 53 kDa. Either or both of FlaA and/or FlaB may be used as a source of type-common antigens for use with the present invention. Another representative type-common antigen includes *H. pylori* urease which is associated with the outer membrane and the periplasmic space of the bacterium. The holoenzyme is a large complex made up of two subunits of 26.5 kDa (UreA) and 61 kDa (UreB), respectively. Type-common epitopes derived from the holoenzyme, either of the subunits, or a combination of the three, can be present as the type-common antigen(s). Another representative type-common antigen that may be present in the lysate or used in further purified form includes the an *H. pylori* heat shock protein known as "hsp60." The DNA and corresponding amino acid sequences for hsp60 are known. See, e.g., International Publication No. WO 93/18150, published 16 September 1993. The full-length hsp60 antigen shown has about 546 amino acids and a molecular weight of about 58 kDa. It is to be understood that the lysate can also include other type-common antigens not specifically described herein.

By a "type-specific *H. pylori* Type I antigen" is meant a polypeptide, as defined below, derived from *H. pylori* Type I which reacts predominantly with antibodies against *H. pylori* Type I, but not with antibodies against *H. pylori* Type II. Representative type-specific *H. pylori* Type I antigens include: *H. pylori* VacA, also known as CT; and *H. pylori* CagA, also known as CAI antigen and tagA; and epitopes from these antigens which are capable of reacting with antibodies against *H. pylori* Type I but not *H. pylori* Type II. Both VacA and CagA are discussed further below. It is to be understood that other type-specific *H. pylori* Type I antigens, not specifically described herein, are also captured by this definition.

The term "polypeptide" when used with reference to a type-common or type-specific *H. pylori* antigen, such as VacA, CagA or any of the other type-specific or type common antigens described above, refers to a VacA, CagA etc., whether native, recombinant or synthetic, which is derived from any of the various *H. pylori* strains. In the case of type-specific *H. pylori* Type I antigens, the polypeptide will be derived from an *H. pylori* Type I strain. In the case of a type-common antigen, the polypeptide may be derived from either of *H*. *pylori* Type I or Type II. The polypeptide need not include the full-length amino acid sequence of the reference molecule but can include only so much of the molecule as necessary in order for the polypeptide to react with the appropriate *H. pylori* antibodies. Thus, only one or few epitopes of the reference molecule need be present. Furthermore, the polypeptide may comprise a fusion protein between the full-length reference molecule or a fragment of the reference molecule, and another protein that does not disrupt the reactivity of the *H. pylori* polypeptide. It is readily apparent that the polypeptide may therefore comprise the full-length sequence, fragments, truncated and partial sequences, as well as analogs and precursor forms of the reference molecule. The term also intends deletions, additions and substitutions to the reference sequence, so long as the polypeptide retains the ability to react with *H. pylori* antibodies.

In this regard, particularly preferred substitutions will generally be conservative in nature, i.e., those substitutions that take place within a family of amino acids that are related in their side chains. Specifically, amino acids are generally divided into four families: (1) acidic -- aspartate and glutamate; (2) basic -- lysine, arginine, histidine; (3) non-polar -- alanine, valine, leucine, isoleucine, proline, phenylalanine, methionine, tryptophan; and (4) uncharged polar -- glycine, asparagine, glutamine, cystine, serine threonine, tyrosine. Phenylalanine, tryptophan, and tyrosine are sometimes classified as aromatic amino acids. For example, it is reasonably predictable that an isolated replacement of leucine with isoleucine or valine, an aspartate with a glutamate, a threonine with a serine, or a similar conservative replacement of an amino acid with a structurally related amino acid, will not have a major effect on the biological activity. Proteins having substantially the same amino acid sequence as the reference molecule, but possessing minor amino acid substitutions that do not substantially affect the antibody binding capabilities of the protein, are therefore within the definition of the reference polypeptide.

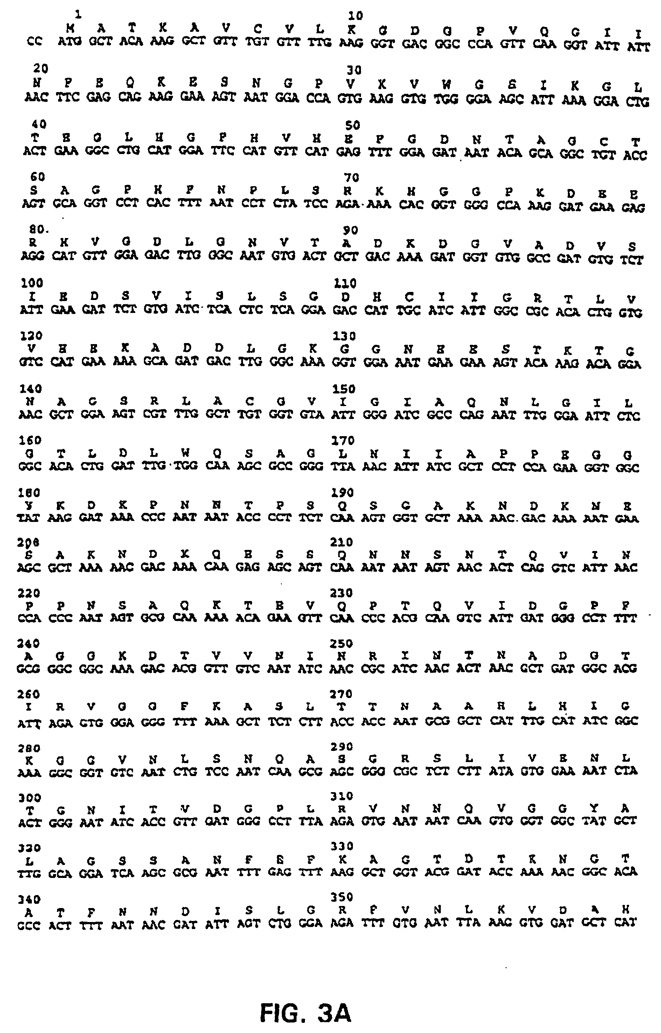

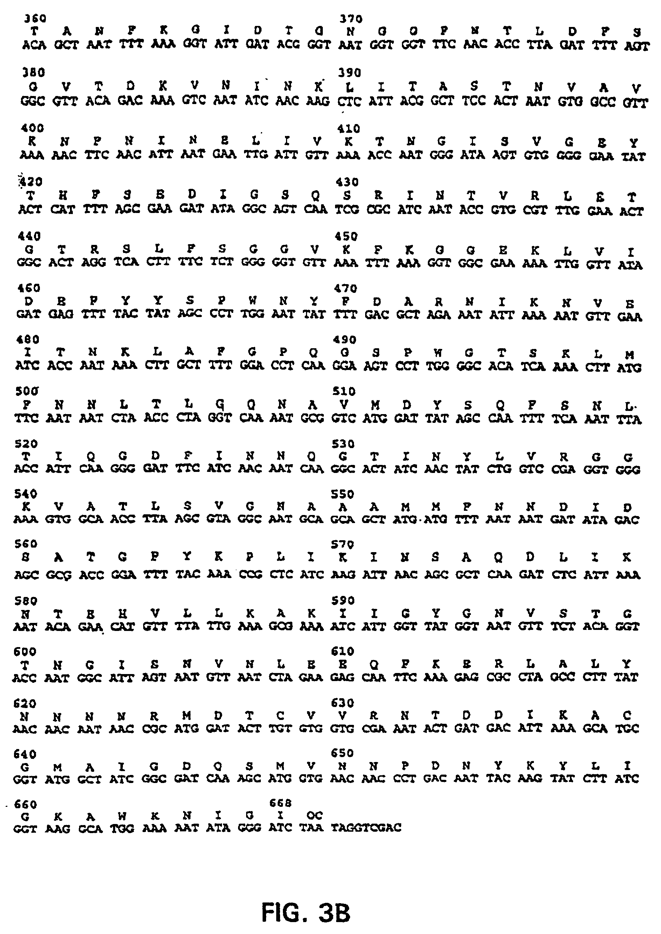

By "VacA polypeptide" is meant a polypeptide as defined above which is derived from the antigen known as the *H. pylori* Type I Cytotoxin and which reacts predominantly with antibodies against *H. pylori* Type I, but not *H. pylori* Type II. The VacA protein induces vacuolization in epithelial cells in tissue culture and causes extensive tissue damage and ulceration when administered orally to mice. The DNA and corresponding amino acid sequences for VacA are known and reported in, e.g., International Publication No. WO 93/18150, published 16 September 1993. The gene for the VacA polypeptide encodes a precursor of about 140 kDa that is processed to an active molecule of about 90-100 kDa. This molecule, in turn, is slowly proteolytically cleaved to generate two fragments that copurify with the intact 90 kDa molecule. See, Telford et al., *Trends in Biotech.* (1994) 12:420-426. Thus, the definition of "VacA polypeptide" as used herein includes the precursor protein, as well as the processed active molecule, proteolytic fragments thereof or portions or muteins thereof, which retain specific reactivity with antibodies present in a biological sample from an individual with *H*. *pylori* Type I infection. For example, the VacA polypeptide depicted in Figures 3A-3B and used in assays described herein includes a VacA fragment from Gly-311 to Ile-819, inclusive, of the full-length molecule, fused by a linker sequence of five amino acids to 154 amino acids of human SOD to facilitate recombinant expression.

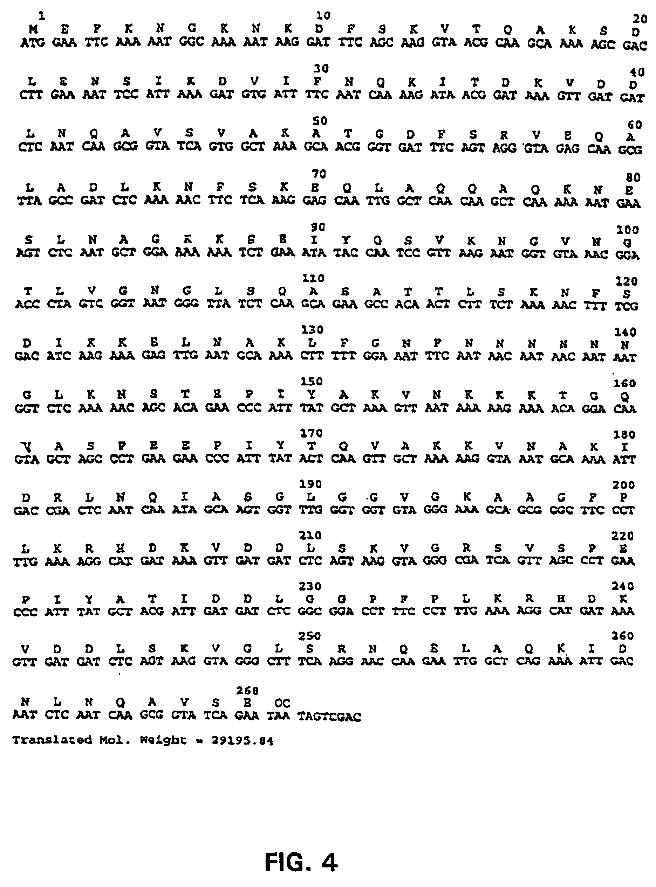

By "CagA polypeptide" is meant a polypeptide as defined above which is derived from the *H*. *pylori* Type I cytotoxin associated immunodominant antigen and which reacts predominantly with antibodies against *H. pylori* Type I, but not *H*. *pylori* Type II. CagA is expressed on the bacterial surface. The DNA and corresponding amino acid sequences for CagA are known. See, e.g., International Publication No. WO 93/18150, published 16 September 1993. The full-length CagA antigen described therein includes about 1147 amino acids with a predicted molecular weight of about 128 kDa. The native protein shows interstrain size variability due to the presence of a variable number of repeats of a 102 bp DNA segment that encodes repeats of a proline-rich amino acid sequence. See, Covacci et al., *Proc*. *Natl. Acad. Sci. USA* (1993) 90:5791-5795. Accordingly, the reported molecular weight of CagA ranges from about 120-135 kDa. Hence, the definition of "CagA polypeptide" as used herein includes any of the various CagA variants, fragments thereof and muteins thereof, which retain the ability to react with antibodies in a biological sample from an individual with *H*. *pylori* Type I infection but does not substantially react with antibodies generated against *H. pylori* Type II. For example, the CagA polypeptide depicted in Figure 4 and used in assays described herein is a truncated protein of 268 amino acids and includes Glu-748 to Glu-1015, inclusive, of the full-length molecule.

By "epitope" is meant a site on an antigen to which specific B cells and T cells respond. The term is also used interchangeably with "antigenic determinant" or "antigenic determinant site." An epitope can comprise 3 or more amino acids in a spatial conformation unique to the epitope. Generally, an epitope consists of at least 5 such amino acids and, more usually, consists of at least 8-10 such amino acids. Methods of determining spatial conformation of amino acids are known in the art and include, for example, x-ray crystallography and 2-dimensional nuclear magnetic resonance. Furthermore, the identification of epitopes in a given protein is readily accomplished using techniques well known in the art, such as by the use of hydrophobicity studies and by site-directed serology. See, also, Geysen et al., *Proc. Natl. Acad. Sci. USA* (1984) 81:3998-4002 (general method of rapidly synthesizing peptides to determine the location of immunogenic epitopes in a given antigen); U.S. Patent No. 4,708,871 (procedures for identifying and chemically synthesizing epitopes of antigens); and Geysen et al., *Molecular Immunology* (1986) 23:709-715 (technique for identifying peptides with high affinity for a given antibody). Antibodies that recognize the same epitope can be identified in a simple immunoassay showing the ability of one antibody to block the binding of another antibody to a target antigen.

A "purified" protein or polypeptide is a protein which is recombinantly or synthetically produced, or isolated from its natural host, such that the amount of protein present in a composition is substantially higher than that present in a crude preparation. In general, a purified protein will be at least about 50% homogeneous and more preferably at least about 80% to 90% homogeneous.

As used herein, a "biological sample" refers to a sample of tissue or fluid isolated from an individual, including but not limited to, for example, blood, plasma, serum, fecal matter, urine, bone marrow, bile, spinal fluid, lymph fluid, samples of the skin, external secretions of the skin, respiratory, intestinal, and genitourinary tracts, samples derived from the gastric epithelium and gastric mucosa, tears, saliva, milk, blood cells, organs, biopsies and also samples of *in vitro* cell culture constituents including but not limited to conditioned media resulting from the growth of cells and tissues in culture medium, e.g., recombinant cells, and cell components.

As used herein, the terms "label" and "detectable label" refer to a molecule capable of detection, including, but not limited to, radioactive isotopes, fluorescers, chemiluminescers, enzymes, enzyme substrates, enzyme cofactors, enzyme inhibitors, chromophores, dyes, metal ions, metal sols, ligands (e.g., biotin or haptens) and the like. The term "fluorescer" refers to a substance or a portion thereof which is capable of exhibiting fluorescence in the detectable range. Particular examples of labels which may be used under the invention include fluorescein, rhodamine, dansyl, umbelliferone, Texas red, luminol, acradimum esters, NADPH and α-β-galactosidase.

### II. Modes of Carrying Out the Invention

The present invention is based on the discovery of novel diagnostic methods for accurately detecting *H. pylori* infection and for discriminating between *H. pylori* Type I and *H. pylori* Type II infection. The methods utilize one or more *H*. *pylori* type-common antigens, either purified or present in lysates derived from the bacterium, as well as purified type-specific *H. pylori* antigens. The use of both type-common and type-specific antigens reduces the incidence of false positive results. The methods can be practiced in a simple one-step assay format which allows for both detection of infection, as well as identification of the type of infection present, in a single assay. The method can also be practiced in two steps wherein the sample is first reacted with the *H. pylori* type-common antigens and if positive, reacted with one or more type-specific Type I antigens. The methods can also employ type-specific Type II antigens.

More particularly, the use of the *H. pylori* type-common antigens allows the diagnosis of *H. pylori* infection in general. The presence of one or more type-specific antigens allows determination of the bacterial type, i.e., whether the infection is caused by *H. pylori* Type I and/or *H. pylori* Type II. Due to the presence of the *H. pylori* type-common antigens, positive results will occur even in untypable samples. Hence, the incidence of false negatives is reduced. Furthermore, if *H. pylori* Type I infection is present, the individual can be administered antibiotics to treat or prevent type B gastritis, peptic ulcers, and gastric tumors. Furthermore, the assays described herein are useful for monitoring the course of treatment in a subject to determine whether antibiotic therapy is effective.

The antigens for use in the subject diagnostic techniques can be produced using a variety of techniques.

For example, the type-common antigens can be provided in a lysate that can be obtained using methods well known in the art. Generally, such methods entail extracting type-common proteins from either *H*. *pylori* Type I or Type II bacteria using sonication, pressure disintegration, detergent extraction, fractionation, and the like. Type-common antigens present in such lysates can be further purified if desired, using standard purification techniques. *H. pylori* strains for use in such methods are readily available from several sources including the American Type Culture Collection (ATCC, Rockville, MD.). For example, ATCC strain designations NCTC 11637, 11639 and 11916, will find use as a source of the lysate. Other useful strains are known in the art.

The type-specific *H. pylori* Type I antigens can also be obtained using standard purification techniques. In this regard, particular antigens can be isolated from Type I *H. pylori* ulcer-producing strains using standard purification techniques such as column chromatography, electrophoresis, HPLC, immunoadsorbent techniques, affinity chromatography and immunoprecipitation. See, e.g., International Publication No. WO 96/12965, published 2 May 1996, for a description of the purification of several antigens from *H. pylori*. For example, ATCC strain designation NCTC 11916 is a Type I ulcer-producing strain of *H.* *pylori* and can therefore be used as a source for one or more type-specific antigens for use in the subject invention.

The *H*. *pylori* antigens can also be generated using recombinant methods, well known in the art. In this regard, oligonucleotide probes can be devised based on the known sequences of the *H*. *pylori* genome and used to probe genomic or cDNA libraries for *H*. *pylori* genes encoding for the antigens useful in the present invention. The genes can then be further isolated using standard techniques and, if desired, restriction enzymes employed to mutate the gene at desired portions of the full-length sequence.

Similarly, *H. pylori* genes can be isolated directly from bacterial cells using known techniques, such as phenol extraction, and the sequence can be further manipulated to produce any desired alterations. See, e.g., Sambrook et al., *supra,* for a description of techniques used to obtain and isolate DNA. Finally, the genes encoding the *H. pylori* antigens can be produced synthetically, based on the known sequences. The nucleotide sequence can be designed with the appropriate codons for the particular amino acid sequence desired. In general, one will select preferred codons for the intended host in which the sequence will be expressed. The complete sequence is generally assembled from overlapping oligonucleotides prepared by standard methods and assembled into a complete coding sequence. See, e.g., Edge, *Nature* (1981) 292:756; Nambair et al., *Science* (1984) 223:1299; Jay et al., *J*. *Biol. Chem.* (1984) 259:6311.

Once coding sequences for the desired polypeptides have been isolated or synthesized, they can be cloned into any suitable vector or replicon for expression in a variety of systems, including insect, mammalian, bacterial, viral and yeast expression systems, all well known in the art. In particular, host cells are transformed with expression vectors which include control sequences operably linked to the desired coding sequence.

The control sequences will be compatible with the particular host cell used. For example, typical promoters for mammalian cell expression include the SV40 early promoter, mouse mammary tumor virus LTR promoter, adenovirus major late promoter (Ad MLP), and herpes simplex virus promoter, among others. Other non-viral promoters, such as a promoter derived from the murine metallothionein gene, will also find use in mammalian constructs. Mammalian expression may be either constitutive or regulated (inducible), depending on the promoter. Typically, transcription termination and polyadenylation sequences will also be present, located 3' to the translation stop codon. Examples of transcription terminator/polyadenylation signals include those derived from SV40 (Sambrook et al., *supra*). Introns, containing splice donor and acceptor sites, may also be designed into the constructs of the present invention.

Enhancer elements can also be used in the mammalian constructs to increase expression levels. Examples include the SV40 early gene enhancer (Dijkema et al., *EMBO J.* (1985) 4:761) and the enhancer/promoters derived from the long terminal repeat (LTR) of the Rous Sarcoma Virus (Gorman et al., *Proc. Natl*. *Acad. Sci. USA* (1982b) 79:6777) and human cytomegalovirus (Boshart et al., *Cell* (1985) 41:521). A leader sequence can also be present which includes a sequence encoding a signal peptide, to provide for the secretion of the foreign protein in mammalian cells. Preferably, there are processing sites encoded between the leader fragment and the gene of interest such that the leader sequence can be cleaved either *in vivo* or *in vitro*. The adenovirus tripartite leader is an example of a leader sequence that provides for secretion of a foreign protein in mammalian cells.

Once complete, the mammalian expression vectors can be used to transform any of several mammalian cells. Methods for introduction of heterologous polynucleotides into mammalian cells are known in the art and include dextran-mediated transfection, calcium phosphate precipitation, polybrene mediated transfection, protoplast fusion, electroporation, encapsulation of the polynucleotide(s) in liposomes, and direct microinjection of the DNA into nuclei.

Mammalian cell lines available as hosts for expression are also known and include many immortalized cell lines available from the American Type Culture Collection (ATCC), including but not limited to, Chinese hamster ovary (CHO) cells, HeLa cells, baby hamster kidney (BHK) cells, monkey kidney cells (COS), human hepatocellular carcinoma cells (e.g., Hep G2), as well as others.

The constructs of the present invention can also be expressed in yeast. Control sequences for yeast vectors are known in the art and include promoters such as alcohol dehydrogenase (ADH) (EP Publication No. 284,044), enolase, glucokinase, glucose-6-phosphate isomerase, glyceraldehyde-3-phosphate-dehydrogenase (GAP or GAPDH), hexokinase, phosphofructokinase, 3-phosphoglycerate mutase, and pyruvate kinase (PyK) (EP Publication No. 329,203). The yeast *PHO5* gene, encoding acid phosphatase, also provides useful promoter sequences (Myanohara et al., *Proc. Natl. Acad. Sci. USA* (1983) 80:1). In addition, synthetic promoters which do not occur in nature also function as yeast promoters. For example, upstream activating sequences (UAS) of one yeast promoter may be joined with the transcription activation region of another yeast promoter, creating a synthetic hybrid promoter. Examples of such hybrid promoters include the ADH regulatory sequence linked to the GAP transcription activation region (U.S. Patent Nos. 4,876,197 and 4,880,734). Other examples of hybrid promoters include promoters which consist of the regulatory sequences of either the *ADH2*, *GAL4*, *GAL10,* or *PHO5* genes, combined with the transcriptional activation region of a glycolytic enzyme gene such as *GAP* or *PyK* (EP Publication No. 164,556). Furthermore, a yeast promoter can include naturally occurring promoters of non-yeast origin that have the ability to bind yeast RNA polymerase and initiate transcription.

Other control elements which may be included in the yeast expression vectors are terminators (e.g., from *GAPDH* and from the enolase gene (Holland, *J. Biol. Chem.* (1981) 256:1385), and leader sequences which encode signal sequences for secretion. DNA encoding suitable signal sequences can be derived from genes for secreted yeast proteins, such as the yeast invertase gene (EP Publication No. 012,873; JPO Publication No. 62,096,086) and the α-factor gene (U.S. Patent Nos. 4,588,684, 4,546,083 and 4,870,008; EP Publication No. 324,274; PCT Publication No. WO 89/02463). Alternatively, leaders of non-yeast origin, such as an interferon leader, also provide for secretion in yeast (EP Publication No. 060,057)

Expression and transformation vectors, either extrachromosomal replicons or integrating vectors, have been developed for transformation into many yeasts. For example, expression vectors have been developed for, *inter alia,* the following yeasts: *Saccharomyces cerevisiae* (Hinnen et al., *Proc. Natl. Acad. Sci. USA* (1978) 75:1929; Ito et al., *J.* *Bacteriol*. (1983) 153:163); *Saccharomyces carlsbergeneis;* Candida *albicans* (Kurtz et al., *Mol. Cell*. *Biol.* (1986) 6:142); *Candida maltosa* (Kunze et al., *J*. *Basic Microbiol.* (1985) 25:141); *Hansenula polymorpha* (Gleeson et al., *J. Gen. Microbiol.* (1986) 132:3459; Roggenkamp et al., *Mol. Gen. Genet.* (1986) 202:302); *Kluyveromyces fragilis* (Das et al., *J. Bacteriol.* (1984) 158:1165); *Kluyveromyces lactis* (De Louvencourt et al., *J. Bacteriol.* (1983) 154:737; Van den Berg et al., *Bio*/*Technology* (1990) 8:135); *Pichia guillerimondii* (Kunze et al., *J. Basic Microbiol.* (1985) 25:141); *Pichia pastoris* (Cregg et al., *Mol. Cell. Biol.* (1985) 5:3376; U.S. Patent Nos. 4,837,148 and 4,929,555); *Schizosaccharomyces pombe* (Beach and Nurse, Nature (1981) 300:706); and *Yarrowia lipolytica* (Davidow et al., *Curr. Genet.* (1985) 10:380471; Gaillardin et al., *Curr. Genet.* (1985) 10:49).

Methods of introducing exogenous DNA into yeast hosts are well known in the art, and typically include either the transformation of spheroplasts or of intact yeast cells treated with alkali cations.

Bacterial expression systems can also be used with the present constructs. Control elements for use in bacteria include promoters, optionally containing operator sequences, and ribosome binding sites. Useful promoters include sequences derived from sugar metabolizing enzymes, such as galactose, lactose (*lac*) and maltose. Additional examples include promoter sequences derived from biosynthetic enzymes such as tryptophan (*trp*), the b-lactamase (*bla*) promoter system, bacteriophage lambda PL, and T5. In addition, synthetic promoters, such as the tac promoter (U.S. Patent No. 4,551,433), which do not occur in nature also function as in bacterial host cells.

The foregoing systems are particularly compatible with *E*. *coli*. However, numerous other systems for use in bacterial hosts such as *Bacillus spp*., *Streptococcus spp.,* and *Streptomyces spp.,* among others, are also known. Methods for introducing exogenous DNA into these hosts typically include the use of CaCl₂ or other agents, such as divalent cations and DMSO. DNA can also be introduced into bacterial cells by electroporation.

Other systems for expression of the desired antigens include insect cells and vectors suitable for use in these cells. The systems most commonly used are derived from the baculovirus *Autographa californica* polyhedrosis virus (AcNPV). Generally, the components of the expression system include a transfer vector, usually a bacterial plasmid, which contains both a fragment of the baculovirus genome, and a convenient restriction site for insertion of the heterologous gene or genes to be expressed; a wild type baculovirus with a sequence homologous to the baculovirus-specific fragment in the transfer vector (this allows for the homologous recombination of the heterologous gene into the baculovirus genome); and appropriate insect host cells and growth media.

Promoters for use in the vectors are typically derived from structural genes, abundantly transcribed at late times in a viral infection cycle. Examples include sequences derived from the gene encoding the viral polyhedron protein, Friesen et al., (1986) "The Regulation of Baculovirus Gene Expression" in: *The Molecular Biology of Baculoviruses* (ed. Walter Doerfler); EP Publication Nos. 127,839 and 155,476; and the gene encoding the p10 protein Vlak et al., *J. Gen. Viral.* (1988) 69:765. The plasmid usually also contains the polyhedrin polyadenylation signal (Miller et al., *Ann. Rev. Microbiol.* (1988) 42:177) and a procaryotic ampicillin-resistance (*amp*) gene and origin of replication for selection and propagation in *E. coli.* DNA encoding suitable signal sequences can also be included and is generally derived from genes for secreted insect or baculovirus proteins, such as the baculovirus polyhedrin gene (Carbonell et al., *Gene* (1988) 73:409), as well as mammalian signal sequences such as those derived from genes encoding human α-interferon, Maeda et al., *Nature* (1985) 315:592; human gastrin-releasing peptide, Lebacq-Verheyden et al., *Molec. Cell. Biol.* (1988) 8:3129; human IL-2, Smith et al., *Proc. Natl*. *Acad. Sci. USA* (1985) 82:8404; mouse IL-3, (Miyajima et al., *Gene* (1987) 58:273; and human glucocerebrosidase, Martin et al., DNA (1988) 7:99.

Currently, the most commonly used transfer vector for introducing foreign genes into AcNPV is pAc373. Many other vectors, known to those of skill in the art, have also been designed. These include, for example, pVL985 (which alters the polyhedrin start codon from ATG to ATT, and which introduces a *BamHI* cloning site 32 bps downstream from the ATT; see Luckow and Summers, *Virology* (1989) 17:31).

The desired DNA sequence is inserted into the transfer vector, using known techniques (see, Summers and Smith, *supra;* Smith et al., *Mol. Cell. Biol.* (1983) 3:2156; and Luckow and Summers (1989) and an insect cell host is cotransformed with the heterologous DNA of the transfer vector and the genomic DNA of wild type baculovirus--usually by cotransfection. The vector and viral genome are allowed to recombine. The packaged recombinant virus is expressed and recombinant plaques are identified and purified. Materials and methods for baculovirus/insect cell expression systems are commercially available in kit form from, for example, Invitrogen, San Diego CA ("MaxBac" kit). These techniques are generally known to those skilled in the art and fully described in Summers and Smith, *Texas Agricultural Experiment Station Bulletin No. 1555* (1987) (hereinafter "Summers and Smith").

Recombinant baculovirus expression vectors have been developed for infection into several insect cells. For example, recombinant baculoviruses have been developed for, *inter alia*: *Aedes aegypti, Autographs californica*, *Bombyx mori, Drosophila melanogaster, Spodoptera frugiperda,* and *Trichoplusia ni.*

It is often desirable that the polypeptides prepared using the above systems be fusion polypeptides. As with nonfusion proteins, these proteins may be expressed intracellularly or may be secreted from the cell into the growth medium.

Furthermore, plasmids can be constructed which include a chimeric gene sequence, encoding e.g., multiple type-specific *H. pylori* Type I antigens or multiple type-common antigens. The gene sequences can be present in a dicistronic gene configuration. Additional control elements can be situated between the various genes for efficient translation of RNA from the distal coding region. Alternatively, a chimeric transcription unit having a single open reading frame encoding the multiple antigens can also be constructed. Either a fusion can be made to allow for the synthesis of a chimeric protein or alternatively, protein processing signals can be engineered to.provide cleavage by a protease, thus allowing liberation of the two or more proteins derived from translation of the template RNA. The processing protease may also be expressed in this system either independently or as part of a chimera with the antigen coding region(s).

Viral systems, such as a vaccinia based infection/transfection system, as described in Tomei et al., *J. Virol.* (1993) 67:4017-4026 and Selby et al., *J. Gen. Virol*. (1993) 74:1103-1113, will also find use with the present invention. In this system, cells are first transfected in vitro with a vaccinia virus recombinant that encodes the bacteriophage T7 RNA polymerase. This polymerase displays exquisite specificity in that it only transcribes templates bearing T7 promoters. Following infection, cells are transfected with the DNA of interest, driven by a T7 promoter. The polymerase expressed in the cytoplasm from the vaccinia virus recombinant transcribes the transfected DNA into RNA which is then translated into protein by the host translational machinery. The method provides for high level, transient, cytoplasmic production of large quantities of RNA and its translation product(s).

Depending on the expression system and host selected, the antigens of the present invention are produced by growing host cells transformed by an expression vector under conditions whereby the antigen of interest is expressed. The antigen is then isolated from the host cells and purified. If the expression system provides for secretion of the antigen, the antigen can be purified directly from the media. If the antigen is not secreted, it is isolated from cell lysates. The selection of the appropriate growth conditions and recovery methods are within the skill of the art.

The *H. pylori* antigens may also be produced by chemical synthesis such as by solid phase or solution peptide synthesis, using methods known to those skilled in the art. Chemical synthesis of peptides may be preferable if the antigen in question is relatively small. See, e.g., J. M. Stewart and J. D. Young, *Solid Phase Peptide Synthesis,* 2nd Ed., Pierce Chemical Co., Rockford, IL (1984) and G. Barany and R. B. Merrifield, The *Peptides: Analysis, Synthesis, Biology,* editors E. Gross and J. Meienhofer, Vol. 2, Academic Press, New York, (1980), pp. 3-254, for solid phase peptide synthesis techniques; and M. Bodansky, *Principles* of *Peptide Synthesis,* Springer-Verlag, Berlin (1984) and E. Gross and J. Meienhofer, Eds., *The Peptides: Analysis, Synthesis, Biology, supra,* Vol. 1, for classical solution synthesis.

The *H. pylori* type-common and type-specific *H*. *pylori* Type I antigens are used herein as diagnostics to detect the presence of reactive antibodies directed against the bacterium in a biological sample. Furthermore, the antigens can be used to monitor the course of antibiotic therapy by comparing results obtained at the outset of therapy to those obtained during and after a course of treatment. For example, the presence of antibodies reactive with the type-common and/or the type-specific *H. pylori* antigens can be detected using standard electrophoretic and immunodiagnostic techniques, including immunoassays such as competition, direct reaction, or sandwich type assays. Such assays include, but are not limited to, Western blots; agglutination tests; enzyme-labeled and mediated immunoassays, such as ELISAs; biotin/avidin type assays; radioimmunoassays; immunoelectrophoresis; immunoprecipitation, etc. The reactions generally include revealing labels such as fluorescent, chemiluminescent, radioactive, enzymatic labels or dye molecules, or other methods for detecting the formation of a complex between the antigen and the antibody or antibodies reacted therewith.

The aforementioned assays generally involve separation of unbound antibody in a liquid phase from a solid phase support to which antigen-antibody complexes are bound. Solid supports which can be used in the practice of the invention include substrates such as nitrocellulose (e.g., in membrane or microtiter well form); polyvinylchloride (e.g., sheets or microtiter wells); polystyrene latex (e.g, beads or microtiter plates); polyvinylidine fluoride; diazotized paper; nylon membranes; activated beads, magnetically responsive beads, and the like.

Typically, a solid support is first reacted with a solid phase component (e.g., one or more type-common and/or one or more type-specific *H. pylori* Type I antigens) under suitable binding conditions such that the component is sufficiently immobilized to the support. Sometimes, immobilization of the antigen to the support can be enhanced by first coupling the antigen to a protein with better binding properties. Suitable coupling proteins include, but are not limited to, macromolecules such as serum albumins including bovine serum albumin (BSA), keyhole limpet hemocyanin, immunoglobulin molecules, thyroglobulin, ovalbumin, and other proteins well known to those skilled in the art. Other molecules that can be used to bind the antigens to the support include polysaccharides, polylactic acids, polyglycolic acids, polymeric amino acids, amino acid copolymers, and the like. Such molecules and methods of coupling these molecules to the antigens, are well known to those of ordinary skill in the art. See, e.g., Brinkley, M.A., *Bioconjugate Chem.* (1992) 3:2-13; Hashida et al., *J. Appl. Biochem.* (1984) 6:56-63; and Anjaneyulu and Staros, International *J. of Peptide and Protein Res.* (1987) 30:117-124.

After reacting the solid support with the solid phase component, any non-immobilized solid-phase components are removed from the support by washing, and the support-bound component is then contacted with a biological sample suspected of containing ligand moieties (e.g., antibodies toward the immobilized antigens) under suitable binding conditions. After washing to remove any non-bound ligand, a secondary binder moiety is added under suitable binding conditions, where the secondary binder is capable of associating selectively with the bound ligand. The presence of the secondary binder can then be detected using techniques well known in the art.

More particularly, an ELISA method can be used, where the wells of a microtiter plate are coated with the *H. pylori* type-common and/or the type-specific *H. pylori* Type I antigen(s). A biological sample containing or suspected of containing anti-*H*. *pylori* immunoglobulin molecules is then added to the coated wells. In assays where it is desired to use one microtiter plate, a selected number of wells can be coated with, e.g., a first type-specific antigen moiety, a different set of wells coated with a second type-specific antigen moiety and a third set of wells with the *H. pylori* type-common antigen, etc. In the alternative, a series of ELISAs can be run in tandem, wherein individual plates are used for each type-common and type-specific antigen moiety. After a period of incubation sufficient to allow antibody binding to the immobilized antigens, the plate(s) can be washed to remove unbound moieties and a detectably labeled secondary binding molecule added. The secondary binding molecule is allowed to react with any captured sample antibodies, the plate washed and the presence of the secondary binding molecule detected using methods well known in the art.

Thus, in one particular embodiment, the presence of bound anti-type-specific and/or anti-*H*. *pylori* type-common antigen ligands from a biological sample can be readily detected using a secondary binder comprising an antibody directed against the antibody ligands. A number of anti-human immunoglobulin (Ig) molecules are known in the art (e.g., commercially available goat anti-human Ig or rabbit anti-human Ig). Ig molecules for use herein will preferably be of the IgG or IgA type, however, IgM may also be appropriate in some instances. The Ig molecules can be readily conjugated to a detectable enzyme label, such as horseradish peroxidase, glucose oxidase, Beta-galactosidase, alkaline phosphatase and urease, among others, using methods known to those of skill in the art. An appropriate enzyme substrate is then used to generate a detectable signal. In other related embodiments, competitive-type ELISA techniques can be practiced using methods known to those skilled in the art.

Assays can also be conducted in solution, such that the bacterial proteins and antibodies specific for those bacterial proteins form complexes under precipitating conditions. In one particular embodiment, the *H. pylori* type-common and/or the type-specific antigen(s) can be attached to a solid phase particle (e.g., an agarose bead or the like) using coupling techniques known in the art, such as by direct chemical or indirect coupling. The antigen-coated particle is then contacted under suitable binding conditions with a biological sample suspected of containing antibodies for *H. pylori* Type I or Type II. Cross-linking between bound antibodies causes the formation of particle-antigen-antibody complex aggregates which can be precipitated and separated from the sample using washing and/or centrifugation. The reaction mixture can be analyzed to determine the presence or absence of antibody-antigen complexes using any of a number of standard methods, such as those immunodiagnostic methods described above.

In yet a further embodiment, an immunoaffinity matrix can be provided, wherein a polyclonal population of antibodies from a biological sample suspected of containing anti-*H*. *pylori* antibodies is immobilized to a substrate. In this regard, an initial affinity purification of the sample can be carried out using immobilized antigens. The resultant sample preparation will thus only contain anti-*H. pylori* moieties, avoiding potential nonspecific binding properties in the affinity support. A number of methods of immobilizing immunoglobulins (either intact or in specific fragments) at high yield and having good retention of antigen binding activity, are known in the art. Not being limited by any particular method, immobilized protein A or protein G can be used to immobilize immunoglobulins.

Accordingly, once the immunoglobulin molecules have been immobilized to provide an immunoaffinity matrix, the *H. pylori* type-common and type-specific antigens, having separate and distinct labels, are contacted with the bound antibodies under suitable binding conditions. After any non-specifically bound antigen has been washed from the immunoaffinity support, the presence of bound antigen can be determined by assaying for each specific label using methods known in the art.

A particularly preferred method for diagnosing *H. pylori* infection and monitoring the course of treatment using the present invention involves the use of strip immunoblot assay (SIA) techniques, such as those known in the art which combine traditional Western and dot blotting techniques, e.g., the RIBA® (Chiron Corp., Emeryville, CA) test. In these assays, the *H*. *pylori* type-common and type-specific *H*. *pylori* Type I antigens are immobilized as individual, discrete portions, e.g., as bands or dots, on a membranous support. Thus, by "discretely immobilized" on a membrane support is meant that the antigens are present as separate components and are not mixed, such that reactivity or lack thereof with each of the antigens present can be assessed. A biological sample suspected of containing antibodies to *H. pylori* antigens is then reacted with the test membrane. Visualization of anti-*H. pylori* reactivity in the biological sample is accomplished using anti-human IgG enzyme-conjugates in conjunction with a colorimetric enzyme substrate. Internal controls, such as human IgM and human IgG, can also be present on the strip. The assay can be performed manually or used in an automated format.

Generally, the type-specific antigens, such as VacA and CagA, are applied to the strip in a concentration of about .5 to about 5 µg/ml, more preferably about .5 to 3 µg/ml and most preferably about 1-2 µg/ml. Alternatively, two concentrations of antigen can be present, such as a low concentration and a high concentration, to provide a strip that can be used both for diagnosis of infection, as well as to monitor the response to treatment. Thus, for example, VacA and/or CagA can be provided in a concentration as specified above, as well as in one or more additional bands, in a concentration of about .005-.4 µg/ml, more preferably about .008-3 µg/ml and most preferably about .1-.2 µg/ml. The type-common antigen, e.g., the *H. pylori* lysate, can be applied at a concentration of about .25-2 µg/ml, more preferably about .25-1.5 µg/ml and most preferably about .5-1 µg/ml. It is readily apparent that the concentration of antigen to be applied to the test strip will vary depending on the specific antigen used and can be readily determined by one of skill in the art.

The Ig controls, such as IgG, can be present in a single concentration, or in two concentrations, one low and one high. For example, IgG can be present in a concentration of about 50-250 ng/ml, more preferably about 75-200 ng/ml and most preferably about 100-185 ng/ml. A higher concentration of IgG can also be present along with the low concentration of IgG, to provide another internal control, such as at a concentration of about 400-1200 ng/ml, more preferably about 450-1000 ng/ml and most preferably about 500-950 ng/ml.

The above-described assay reagents, including the *H. pylori* lysate and type-specific antigens, optionally immobilized on a solid support, can be provided in kits, with suitable instructions and other necessary reagents, in order to conduct immunoassays as described above. The kit can also contain, depending on the particular immunoassay used, suitable labels and other packaged reagents and materials (i.e. wash buffers and the like). Standard immunoassays, such as those described above, can be conducted using these kits.

### III. Experimental

Below are examples of specific embodiments for carrying out the present invention. The examples are offered for illustrative purposes only, and are not intended to limit the scope of the present invention in any way.

Efforts have been made to ensure accuracy with respect to numbers used (e.g., amounts, temperatures, etc.), but some experimental error and deviation should, of course, be allowed for.

### Example 1

### Production of an H. pylori VacA Polypeptide

A VacA polypeptide was produced recombinantly as a fusion protein of 71.2 kDa which included 154 amino acids of human superoxide dismutase (SOD) (Hallewell et al., *Nucl. Acids Res.* (1985) 13:2017-2134), a linker of five amino acids (Asn-Leu-Gly-Ile-Leu) and the VacA amino acid sequence Gly-311 through Ile-819 of *H. pylori CCUG17874* (Covacci et al., *Proc. Natl. Acad. Sci. USA* (1993) 90:5791-5795; Telford et al., *J. Exp. Med.* (1994) 179:1653-1658).

In particular, DNA encoding for the truncated VacA protein was synthesized by PCR and fused in frame with DNA sequences coding for SOD. The glucose regulated ADH2/GAPDH promoter (Cousens et al., Gene (1984) 61:265-275) was then incorporated at the 5'-end of the amplified fragment and the resulting cassette was cloned into the yeast expression vector pBS24.1 (Pichuantes et al., *Protein Eng*., *Principle and Prac.* (1996) 5:129-161). This vector contains 2µ and inverted repeat (IR) sequences for autonomous replication in yeast, the α-factor terminator to ensure transcription termination, the *leu2-d* and URA3 yeast genes for selection, and the β-lactamase gene and the ColE1 origin of replication for selection and propagation in *E*. *coli.* High expression levels of the *H. pylori* VacA recombinant protein were obtained in *Saccharomyces cerevisiae JSC310 (Mat a, leu2, ura3-52, prb1-1122, pep4-3*, *prc1-407, ::pDM15 (G418*^{*R*}*), [cir*^{*0*}*])* as evidenced by Coomassie-blue staining and immunoblot analysis of yeast proteins separated by SDS-PAGE. The nucleotide and corresponding amino acid sequence of the VacA recombinant protein is shown in Figures 3A-3B.

The VacA recombinant protein was purified from yeast cells harvested several hours after depletion of glucose from the medium. This condition is needed to activate the ADH2/GAPDH promoter and trigger production of the foreign protein (Pichuantes et al., *J. Biol. Chem.* (1990) 265:13890-13898). Cells were broken with glass beads in a Dynomill using a lysis buffer containing 50mM Tris-HCl (pH 8.0), 150mM NaCl, 1mM EDTA, 1mM PMSF. The protein was recovered from the insoluble fraction (obtained by centrifugation at 48,400 x g for 30 minutes) with increasing amounts of urea (1M to 3M) in lysis buffer. After centrifugation at 48,400 x g for 30 minutes, the pellet containing the protein of interest was solubilized with lysis buffer containing 4M urea, 50mM DTT, 1N NaOH, while stirring on ice for 30 minutes. After removal of cell debris by centrifugation, the suspension was immediately titrated back to pH 8.0 with 6N NaOH. The supernatant was made 2.3% SDS, boiled for 3 minutes, cooled to room temperature and loaded onto a Sephacryl S-400 gel filtration column (Pharmacia) using a buffer containing PBS, 0.1mM EDTA, 0.1% SDS, pH 7.4. Fractions containing the recombinant VacA protein were pooled and concentrated in an Amicon concentrator (YM-10 membrane). After adjusting SDS to 2.3% and DTT to 50mM, the suspension was loaded back onto the same S-400 column for further fractionation. This procedure yielded a VacA protein >90 pure.

### Example 2

### Production of an H. pylori CagA Polyseptide

A CagA polypeptide of 268 amino acids and having a molecular weight of 29.2 kDa, including amino acids Glu-748 through Glu-1015 of the CagA protein of the Chilean strain of *Helicobacter pylori*, *Chetx-1,* was produced recombinantly as follows. The DNA coding for this truncated CagA protein was synthesized by PCR and the initiation codon ATG was introduced at the 5'-end of the amplified fragment. The ADH2/GAPDH hybrid promoter (Cousens et al., Gene (1984) 61:265-275) was then incorporated at the 5'-end of the PCR-synthesized fragment and the resulting cassette was cloned into the yeast expression vector pBS24.1, essentially as described above. The resulting recombinant plasmid was used to transform *Saccharomyces cerevisiae AD3 (Mat a, leu2*, *ura3-52, prb1-1122, pep4-3, prc1-407, ::pDM15 (G418*^{*R*}*), LEU2(ΔAD), [CIR*^{*0*}*])* and expression of the recombinant antigen was monitored by Coomassie blue staining and immunoblot analysis of yeast proteins fractionated by SDS-PAGE. The nucleotide and corresponding amino acid sequence of the CagA recombinant protein is shown in Figure 4.

The CagA recombinant protein was purified from yeast cells suspended in lysis buffer (50mM Tris-HC1, 150mM NaCl, 1mM EDTA, 1mM PMSF, pH 8.0) and disrupted with glass beads in a Dynomill. After centrifugation at 48,400 x g for 30 minutes, the supernatant was made 4M urea, diluted 1/10 (v/v) with S Sepharose IEC equilibration buffer to OD₂₈₉ = 0.00 and the protein was eluted with a linear gradient of 0 to 0.5M NaCl in equilibration buffer. The elution peak containing the CagA protein was collected and precipitated with 30% ammonium sulfate. After centrifugation at 17,700 x g for 30 minutes, the pellet was discarded and the supernatant was precipitated with a further 10% solid ammonium sulfate under the conditions described above. The pellet obtained after centrifugation at 17,700 x g for 30 minutes was dissolved in a minimum amount of PBS/8M urea, adjusted to 2.3% SDS/50mM DTT, boiled for 3 minutes, cooled to room temperature and loaded onto a Sephacryl S-300 gel filtration column (Pharmacia) using a buffer containing PBS, 0.1mM SDS, 1mM EDTA, pH 7.4. The fractions containing the protein of interest were collected and concentrated in an Amicon concentrator (YM-10 membrane). The concentrated material was made 2.3% SDS/50mM DTT and loaded back onto the same S-300 column for a second fractionation. This procedure yielded a CagA protein >90% pure.

### Example 3

### H. pylori Strip Immunoblot assay (SIA) Using Type-Specific and Type-Common H. pylori Antigens

A. An SIA was done as follows. An *H. pylori* extract containing a mixture of antigens including type-common antigens was obtained from Bioseed, Inc., Hillsborough, CA. Briefly, the extract was prepared from an *H. pylori* bacterium obtained from the American Type Culture Collection (ATCC) having an ATCC strain designation of ATCC 43504. The extract was prepared using detergent extraction and sonication.

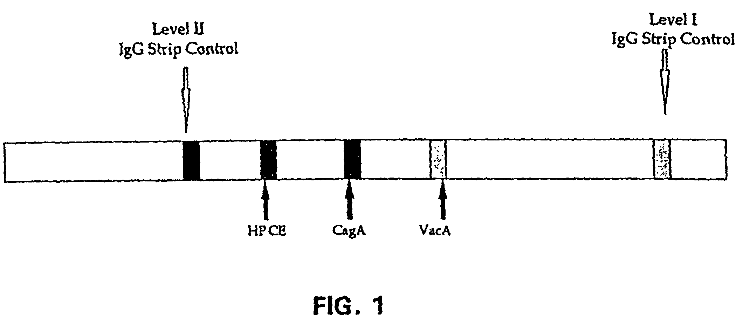

The VacA and CagA type-specific antigens, described in Examples 1 and 2, respectively, were applied in discrete bands to nitrocellulose strips at concentrations of 1-2 µg/ml. The lysate including the type-common antigens was coated as another discrete band at a concentration of 0.5-1 µg/ml onto the same nitrocellulose strips. As internal controls, additional bands contained purified human IgG at a low (100-185 ng/ml) and high (500-925 ng/ml) concentration. Other strips were coated with the antigens and lysate as described above, however, on these strips, the lysate was first enhanced with the VacA and CagA antigens added to the lysate at a concentration of .5-1 µg/ml (VacA) and .5-µg/ml (CagA). Figure 1 is a diagram of a nitrocellulose strip with the antigens as described above.

For the immunoblot assay, strips were processed in a batch fashion with 30 strips per batch. All steps were performed at room temperature. Each strip was numbered and then placed in a separate tube to which was added 1 ml of diluent (phosphate-buffered saline (PBS) with bovine protein stabilizers and detergents, 0.1% sodium azide and 0.05% gentamicin sulfate as preservatives) and 30 µl of a serum sample from an individual known to be infected with *H. pylori* Type I, suspected of being infected with *H. pylori* Type I or a control subject, was applied. The tubes were rocked gently for 4 h, the solution removed by aspiration, and 1 ml of fresh diluent was added to each tube. The tubes were rocked for 30 minutes, the solution removed by aspiration and 1 ml of wash buffer made from wash buffer concentrate (50X) (phosphate-buffered detergent solution with 0.01% thimerosal as a preservative) was added to each tube. The contents of each tube were emptied into a single wash vessel and the strips were washed by swirling for 20 seconds. The wash buffer was decanted and 30 ml of fresh buffer added and the process repeated. Residual solution was removed by aspiration and 20 ml of conjugate solution (peroxidase-labeled goat anti-human IgG (heavy and light chains), with bovine protein stabilizers, containing 0.01% thimerosal as a preservative) was added. The vessel was rotated at 110 rpm for 10 minutes, the conjugate solution was decanted and the wash step was repeated three times. Residual solution was again removed by aspiration and 20 ml of substrate/developer (4-chloro-1-napthol in methanol/phosphate-buffered hydrogen peroxide) added, followed by rotation for 15-20 minutes at 110 rpm. The solution was decanted and the strips were washed twice in distilled water. Developed strips were placed face up on absorbent paper and allowed to dry for 30 minutes in the dark. Strips were then read within 3 h of drying. The intensity of the color of the *H*. *pylori* antigen bands were assigned values ranging from - (0) to 4+ using the following algorithm. The low IgG band is assigned a value of 1+, the high IgG band is assigned a value of 3+. The lysate and VacA and CagA bands are then scored from 0 to 4+ according to how their band intensity compares to that of the IgG control bands.

Samples with any of the *H. pylori* antigen bands scoring 1+ or greater to the lysate band are considered to be positive for *H. pylori* infection. Reactivity of 1+ or greater to the lysate band, in addition to reactivity of 1+ or greater to the CagA and/or VacA band, is considered positive for *H. pylori* Type I infection. Samples showing no reactivity of 1+ or greater to any bands are considered negative.

Using this assay, 29 samples obtained from individuals determined to be positive for *H. pylori* infection by endoscopy, 12 with duodenal ulcers, 9 with gastric ulcers and 8 with gastritis, were tested for reactivity. In another study, 80 individuals known to have duodenal ulcers were tested. The results are shown in Tables 1 and 2, respectively. As can be seen, the assay is highly predictive of *H. pylori* infection.

**Table 1**

| Frequency of Band Reactivity (*H*. *Pylori* Endoscopy Positive Specimens n=29) | | | | |
|---|---|---|---|---|
| | Lysate | Lysate + CagA | Lysate + CagA and/or VacA | CagA and/or VacA |
| D. Ulcer (n=12) | 11/12 91.7% | 10/12 83.3% | 10/12 83.3% | 1/12 8.3% |
| G. Ulcer (n=9) | 9/9 100% | 7/9 77.8% | 8/9 88.9% | 0/9 0.0% |
| Gastritis (n=8) | 6/8 75.0% | 4/8 50.0% | 4/8 50.0% | 1/8 12.5% |

**Table 2**

| Frequency of Band Reactivity in a Duodenal Ulcer Population | | | |
|---|---|---|---|
| Lysate | Lysate + CagA | Lysate + CagA and/or VacA | CagA and/or VacA |
| 78/80 (97.5%) | 71/80 (88.8%) | 75/80 (93.8%) | 2/80 (2.5%) |

The above assay was also used to test samples from individuals undergoing antibiotic therapy for *H*. *pylori* infection. Table 3 shows a comparison between a person responding positively to antibiotic therapy (responder) and one not responding to therapy (non-responder). Samples from the responder were taken at 1, 3, 6, 9 and 12 months and samples from the non-responder taken at 8 and 16 months. As shown in Table 3, reactivity with the antigens decreased with time with the responder but did not significantly change with the non-responder.

**Table 3**

| Therapy Monitoring of *H. pylori* Infections | | | |
|---|---|---|---|
| I. Responder | | | |
| | Lysate | CagA | VacA |
| Prebleed | 3.42 | 1.92 | 0.31 |
| 1M | 2.87 | 1.69 | 0.26 |
| 3M | 2.32 | 1.09 | 0.33 |
| 6M | 2.25 | 0.90 | 0.29 |
| 9M | 2.13 | 0.73 | 0.21 |
| 12M | 1.32 | 0.43 | 0.18 |

| II. Non-Responder | | | |
|---|---|---|---|
| | Lysate | CagA | VacA |
| Prebleed | 3.38 | 1.31 | 2.54 |
| 8M | 3.06 | 0.99 | 2.49 |
| 16M | 2.92 | 0.90 | 2.10 |

In another study, samples were obtained from individuals undergoing three different types of antibiotic regimens, as shown in Table 4. As can be seen, the response rate reported correlated with the results using the assay above for those individuals undergoing the triple and single antibiotic regimens.

**Table 4**

| Therapy Monitoring of *H*. *pylori* Infection | | |
|---|---|---|
| Therapy Regimen | Response Rate Reported | % Responders |
| Triple | 73% | 15/20 (75%) |
| Double | 48% | 11/17 (65%) |
| Single | 19% | 1/5 (20%) |
| Triple = Metronidazole + Bismuth + Amoxicillin | | |
| Double = Metronidazole + Bismuth | | |
| Single = Metronidazole | | |

These two studies show that the assay is useful for determining whether an individual is responding to treatment.

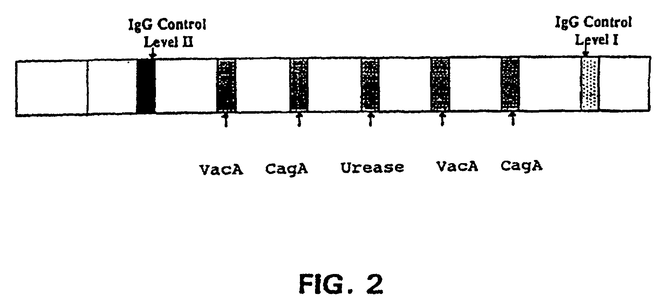

B. Another SIA was done as follows. *H. pylori* urease was applied as a narrow band to nitrocellulose strips at a concentration of 1-2 µg/ml. The VacA antigen, described above, was applied at two concentrations, 1-2 µg/ml and 0.1-0.2 µg/ml, to the same strips. The CagA type-specific antigen was also applied to the strips at the same two concentrations. As internal controls, additional bands contained purified human IgG at a low (100-185 ng/ml) and high (500-925 ng/ml) concentration. Figure 2 is a diagram of a nitrocellulose strip with the antigens as described above.

The assay was performed as described above.

The intensity of the color of the *H. pylori* antigen bands were assigned values, also as described above.

Thus, novel methods for detecting *H. pylori* infection as well as distinguishing between *H. pylori* Type I and Type II, and for monitoring the course of treatment, are disclosed.

## Claims

1. A method of detecting *Helicobacter pylori* infection in a subject comprising:
(a) providing a biological sample from the subject; and
(b) reacting said biological sample with one or more *H.pylori* type-common antigens and reacting the biological sample with one or more purified type-specific *H.pylori* Type I antigens, under conditions which allow *H.pylori* antibodies, when present in the biological sample, to bind with said type-common antigens and/or said type-specific antigen(s),
thereby detecting the presence or absence of *H.pylori* infection in the subject.

2. The method of claim 1, wherein said *H.pylori* infection is *H.pylori* Type I.

3. The method of claim 1, wherein said *H.pylori* infection is *H.pylori* Type II.

4. The method of any preceding claim further comprising:
(c) removing unbound antibodies;
(d) providing one or more moieties capable of associating with said bound antibodies; and
(e) detecting the presence or absence of said one or more moieties,
thereby detecting the presence or absence of *H.pylori* infection.

5. The method of claim 4, wherein said one or more moieties comprises a detectably labeled anti-human immunoglobulin antibody.

6. The method of claim 5 wherein the detectable label is a fluorescer or an enzyme.

7. The method of any preceding claim, wherein said one or more type-common antigens and said one or more type-specific antigens are immobilized on a solid support.

8. The method of claim 7 wherein said one or more type-common antigens and said one or more type-specific antigens are immobilized on the same solid support.

9. The method of claim 7 wherein said one or more type-common antigens and said one or more type-specific antigens are immobilized on different solid supports.

10. The method of claim 7, claim 8 or claim 9, wherein the solid support is a nitrocellulose strip.

11. A method for distinguishing between *Helicobacter pylori* Type I and *Helicobacter pylori* Type II infection in a biological sample, said method comprising:
(a) immobilizing an *H.pylori* lysate comprising one or more *H.pylori* type-common antigens on a nitrocellulose strip, and immobilizing one or more purified type-specific *H.pylori* Type I antigens on a nitrocellulose strip;
(b) contacting said nitrocellulose strip from step (a) with said biological sample under conditions which allow anti*-H.pylori* Type I and anti-*H.pylori* Type II antibodies, when present in the biological sample, to bind with *H.pylori* type-common antigens present in said lysate and/or said type-specific *H.pylori* Type I antigen(s);
(c) removing unbound antibodies;
(d) providing a detectably labeled anti-human immunoglobulin antibody; and
(e) detecting the presence or absence of bound anti-human immunoglobulin antibodies in said biological sample, thereby detecting the presence of absence of *H.pylori* Type I and/or *H.pylori* Type II infection.

12. A method of monitoring a subject undergoing therapy for an *Helicobacter pylori* infection comprising:
(a) providing a biological sample from the subject;
(b) immobilizing an *H.pylori* lysate comprising one or more *H.pylori* type-common antigens on a nitrocellulose strip, and immobilizing one or more purified type-specific *H.pylori* Type I antigens on a nitrocellulose strip;
(c) contacting said nitrocellulose strip from step (b) with said biological sample under conditions which allow anti-*H.pylori* Type I and anti-*H.pylori* Type II antibodies, when present in the biological sample, to bind with *H.pylori* type-common antigens present in said lysate and/or said type-specific *H.pylori* Type I antigen(s);
(d) removing unbound antibodies;
(e) providing a detectably labeled anti-human immunoglobulin antibody; and
(f) detecting the presence or absence of bound anti-human immunoglobulin antibodies in said biological sample,
thereby monitoring the course of treatment of the infection.

13. The method of claim 11 or claim 12 wherein said one or more type-common antigens and said one or more type-specific antigens are immobilized on the same nitrocellulose strip.

14. The method of claim 11 or claim 12, wherein said one or more type-common antigens and said one or more type-specific antigens are immobilized on different nitrocellulose strips.

15. The method of any preceding claim, wherein said one or more type-specific antigens is selected from the group consisting of an *H.pylori* Type I VacA polypeptide and an *H.pylori* Type I CagA polypeptide.

16. The method of any preceding claim, wherein said one or more type-common antigens is provided in an *H.pylori* lysate.

17. The method of any preceding claim, wherein said one or more type-common antigens comprises an *H.pylori* urease.

18. The method of any preceding claim, wherein said biological sample is a human serum sample.

19. A membrane support comprising one or more *H.pylori* type-common antigens and one or more purified type-specific *H.pylori* Type I antigens, discretely immobilized thereon.

20. The membrane support of claim 19, wherein said one or more type-specific antigens is selected from the group consisting of an *H.pylori* Type I VacA polypeptide and an *H.pylori* Type I CagA polypeptide.

21. The membrane support of claim 19, wherein said one or more type-common antigens is provided in an *H.pylori* lysate.

22. The membrane support of claim 19, wherein said one or more type-common antigens comprises an *H.pylori* urease.

23. The membrane support of any one of claims 19 to 22, further comprising a human immunoglobulin, discretely immobilized thereon.

24. The membrane support of any one of claims 19 to 22, wherein the membrane support is a nitrocellulose strip.

25. A nitrocellulose support comprising:
(a) an *H.pylori* Type I VacA polypeptide;
(b) an *H.pylori* Type I CagA polypeptide;
(c) an *H.pylori* urease; and
(d) a human IgG,
wherein said *H.pylori* polypeptides and urease, and said human IgG, are immobilized as discrete bands on said nitrocellulose support.

26. A nitrocellulose support comprising:
(a) an *H.pylori* Type I VacA polypeptide;
(b) an *H.pylori* Type I CagA polypeptide;
(c) an *H.pylori* lysate; and
(d) a human IgG,
wherein said *H.pylori* polypeptides and lysate, and said human IgG, are immobilized as discrete bands on said nitrocellulose support.

27. An immunodiagnostic test kit for detecting *H.pylori* infection, said test kit comprising:
(a) one or more *H.pylori* type-common antigens;
(b) one or more purified type-specific *H.pylori* Type I antigens; and
(c) instructions for conducting the immunodiagnostic test.

28. An immunodiagnostic test kit for distinguishing between *H.pylori* Type I and *H.pylori* Type II infection in a biological sample, said test kit comprising:
(a) an *H.pylori* lysate comprising one or more *H.pylori* type-common antigens immobilized on a nitrocellulose strip;
(b) one or more purified type-specific *H.pylori* Type I antigens immobilized on a nitrocellulose strip; and
(c) instructions for conducting the immunodiagnostic test.

29. An immunodiagnostic test kit for monitoring a subject undergoing therapy for an Helicobacter pylori infection, said test kit comprising:
(a) an *H.pylori* lysate comprising one or more *H.pylori* type-common antigens immobilized on a nitrocellulose strip;
(b) one or more purified type-specific *H.pylori* Type I antigens immobilized on a nitrocellulose strip; and
(c) instructions for conducting the immunodiagnostic test.

30. The immunodiagnostic test kit of claim 27, claim 28 or claim 29, wherein said one or more type-specific antigens is selected from the group consisting of an *H.pylori* Type I VacA polypeptide and an *H.pylori* Type I CagA polypeptide.

31. The immunodiagnostic test kit of claim 27, wherein said one or more type-common antigens are provided in an *H.pylori* lysate.

32. The immunodiagnostic test kit of claim 27, wherein said one or more type-common antigens comprises an *H.pylori* urease.

33. The immunodiagnostic test kit of claim 28 or claim 29, wherein said lysate and said one or more type-specific antigens are immobilized on the same nitrocellulose strip.

34. The immunodiagnostic test kit of claim 28 or claim 29, wherein said lysate and said one or more type-specific antigens are immobilized on different nitrocellulose strips.

## Patentansprüche

1. Verfahren zum Nachweis einer *Helicobacter pylori*-Infektion bei einem Patienten, umfassend:
(a) Bereitstellen einer biologischen Probe des Patienten; und
(b) Umsetzen der biologischen Probe mit einem oder mehreren *H.pylori*-Typ-gemeinsamen Antigenen und Umsetzen der biologischen Probe mit einem oder mehreren gereinigten Typ-spezifischen *H.pylori*-Typ-I-Antigenen unter Bedingungen, die *H.pylori*-Antikörpern erlauben, falls in der biologischen Probe vorliegend, mit den Typ-gemeinsamen Antigenen und/oder dem/den Typ-spezifischen Antigen(en) zu binden,
zum Nachweis der Gegenwart oder Abwesenheit von *H.pylori*-Infektion in dem Patienten.

2. Verfahren nach Anspruch 1, wobei die *H.pylori*-Infektion *H.pylori*-Typ-I ist.

3. Verfahren nach Anspruch 1, wobei die *H.pylori*-Infektion *H.pylori*-Typ-II ist.

4. Verfahren nach einem vorangehenden Anspruch, weiterhin umfassend:
(c) Entfernen von ungebundenen Antikörpern;
(d) Bereitstellen von einer oder mehreren Einheiten, die befähigt sind, mit den gebundenen Antikörpern zu assoziieren; und
(e) Nachweisen der Gegenwart oder Abwesenheit der einen oder mehreren Einheit(en),
zum Nachweis der Gegenwart oder Abwesenheit von *H.pylori*-Infektion.

5. Verfahren nach Anspruch 4, wobei die eine oder mehrere Einheit(en) einen nachweisbar markierten Anti-Human-Immunoglobulin-Antikörper umfasst.

6. Verfahren nach Anspruch 5, wobei die nachweisbare Markierung ein Fluoreszenzmittel oder ein Enzym ist.

7. Verfahren nach einem vorangehenden Anspruch, wobei das/die eine oder mehreren Typ-gemeinsame(n) Antigen(e) und das/die eine oder mehreren Typ-spezifische(n) Antigen(e) auf einem festen Träger immobilisiert sind.

8. Verfahren nach Anspruch 7, wobei das/die eine oder mehreren Typ-gemeinsame(n) Antigen(e) und das/die eine oder mehreren Typ-spezifische(n) Antigen(e) auf dem gleichen festen Träger immobilisiert sind.

9. Verfahren nach Anspruch 7, wobei das/die eine oder mehreren Typ-gemeinsame(n) Antigen(e) und das/die eine oder mehreren Typ-spezifische(n) Antigen(e) auf verschiedenen festen Trägem immobilisiert sind.

10. Verfahren nach Anspruch 7, Anspruch 8 oder Anspruch 9, wobei der feste Träger ein Nitrocellulosestreifen ist.

11. Verfahren zum Unterscheiden zwischen *Helicobacter pylori* Typ I- und *Helicobacter pylori* Typ II-Infektion in einer biologischen Probe, wobei das Verfahren umfasst:
(a) Immobilisieren eines *H.pylori*-Lysats, umfassend ein oder mehrere *H.pylori*-Typ-gemeinsame(s) Antigen(e) auf einem Nitrocellulosestreifen und Immobilisieren von einem oder mehreren gereinigten Typ-spezifischen *H.pylori*-Typ-I-Antigen(en) auf einem Nitrocellulosestreifen;
(b) Inkontaktbringen des Nitrocellulosestreifens von Schritt (a) mit der biologischen Probe unter Bedingungen, die Anti-*H.pylori*-Typ I- und Anti-*H.pylori*-Typ II-Antikörpern erlauben, falls in der biologischen Probe vorliegend, mit in dem Lysat vorliegenden *H.pylori*-Typ-gemeinsamen Antigenen und/oder dem/den Typ-spezifischen *H.pylori*-Typ-I-Antigen(en) zu binden;
(c) Entfernen der ungebundenen Antikörper;
(d) Bereitstellen eines nachweisbar, markierten Anti-Human-Immunoglobulin-Antikörpers; und
(e) Nachweisen der Gegenwart oder Abwesenheit von gebundenen Anti-Human-Immunoglobulin-Antikörpern in der biologischen Probe,
wodurch die Gegenwart oder Abwesenheit von *H.pylori*-Typ-I- und/oder *H.pylori*-Typ-II-Infektion nachgewiesen wird.

12. Verfahren zur Überwachung eines Patienten, der eine Therapie gegen eine *Helicobacter pylori*-Infektion vornimmt, umfassend:
(a) Bereitstellen einer biologischen Probe von dem Patienten;
(b) Immobilisieren eines *H.pylori*-Lysats, umfassend ein oder mehrere *H.pylori*-Typ-gemeinsame(s) Antigen(e) auf einem Nitrocellulosestreifen und Immobilisieren des einen oder mehreren gereinigten Typ-spezifischen *H.pylori*-Typ-I-Antigens/(en) auf einem Nitrocellulosestreifen;
(c) Inkontaktbringen des Nitrocellulosestreifens von Schritt (b) mit der biologischen Probe unter Bedingungen, die Anti-*H.pylori*-Typ-I- und Anti-*H.pylori*-Typ-II-Antikörpern erlauben, falls in der biologischen Probe vorliegend, mit in dem Lysat vorliegenden *H.pylori*-Typ-gemeinsamen Antigenen und/oder dem/den Typ-spezifischen *H.pylori*-Typ-I-Antigen(en) zu binden;
(d) Entfernen von ungebundenen Antikörpern;
(e) Bereitstellen eines nachweisbar, markierten Anti-Human-Immunoglobulin-Antikörpers; und
(f) Nachweis der Gegenwart oder Abwesenheit von gebundenen Anti-Human-Immunoglobulin-Antikörpern in der biologischen Probe,
zum Überwachen des Verlaufs der Behandlung der Infektion.

13. Verfahren nach Anspruch 11 oder Anspruch 12, wobei das/die eine oder mehreren Typ-gemeinsame(n) Antigen(e) und das/die eine oder mehreren Typspezifische(n) Antigen(e) auf dem gleichen Nitrocellulosestreifen immobilisiert werden.

14. Verfahren nach Anspruch 11 oder Anspruch 12, wobei das/die eine oder mehreren Typ-gemeinsame(n) Antigen(e) und das/die eine oder mehreren Typspezifische(n) Antigen(e) auf verschiedenen Nitrocellulosestreifen immobilisiert werden.

15. Verfahren nach einem vorangehenden Anspruch, wobei das/die eine oder mehreren Typ-spezifische(n) Antigen(e) aus der Gruppe, bestehend aus *H.pylori*-Typ-I-VacA-Polypeptid und einem *H.pylori*-Typ-I-CagA-Polypeptid, ausgewählt ist/sind.

16. Verfahren nach einem vorangehenden Anspruch, wobei das/die eine oder mehreren Typ-gemeinsame(n) Antigen(e) in einem *H.pylori*-Lysat bereitgestellt wird/werden.

17. Verfahren nach einem vorangehenden Anspruch, wobei das/die eine oder mehreren Typ-gemeinsame(n) Antigen(e) eine *H.pylori*-Urease umfasst /umfassen.

18. Verfahren nach einem vorangehenden Anspruch, wobei die biologische Probe eine Humanserumprobe ist.

19. Membranträger, umfassend ein oder mehrere *H.pylori*-Typ-gemeinsame Antigen(e) und ein oder mehrere gereinigte Typ-spezifische *H.pylori-*Typ-I-Antigen(e), das/die diskret darauf immobilisiert ist/sind.

20. Membranträger nach Anspruch 19, wobei das/die eine oder mehreren Typspezifische(n) Antigen(e) aus der Gruppe, bestehend aus *H.pylori*-Typ-I-VacA-Polypeptid und einem *H.pylori*-Typ-l-CagA-Polypeptid, ausgewählt ist/sind.

21. Membranträger nach Anspruch 19, wobei das/die eine oder mehreren Typgemeinsame(n) Antigen(e) aus einem *H.pylori*-Lysat bereitgestellt ist/sind.

22. Membranträger nach Anspruch 19, wobei das/die eine oder mehreren Typgemeinsame(n) Antigen(e) eine *H.pylori*-Urease umfasst/umfassen.

23. Membranträger nach einem der Ansprüche 19 bis 22, der weiterhin ein diskret darauf immobilisiertes Human-Immunoglobulin umfasst.

24. Membranträger nach einem der Ansprüche 19 bis 22, wobei der Membranträger ein Nitrocellulosestreifen ist.

25. Nitrocelluloseträger, umfassend:
(a) ein *H.pylori*-Typ-I-VacA-Polypeptid;
(b) ein *H.pylori*-Typ-I-CagA-Polypeptid;
(c) eine *H.pylori*-Urease; und
(d) ein Human-IgG,
wobei die *H.pylori*-Polypeptide und -Urease und das Human-IgG als diskrete Banden auf dem Nitrocelluloseträger immobilisiert sind.

26. Nitrocelluloseträger, umfassend:
(a) ein *H.pylori*-Typ-I-VacA-Polypeptid;
(b) ein *H.pylori*-Typ-I-CagA-Polypeptid;
(c) ein *H.pylori*-Lysat; und
(d) ein Human-IgG,
wobei die *H.pylori*-Polypeptide und das -Lysat und das Human-IgG als diskrete Banden auf dem Nitrocelluloseträger immobilisiert sind.

27. Immunodiagnostisches Test-Kit zum Nachweis von *H.pylori*-Infektion, wobei das Test-Kit umfasst:
(a) ein oder mehrere *H.pylori*-Typ-gemeinsames/e Antigen(e);
(b) ein oder mehrere gereinigte Typ-spezifisches/e *H.pylori*-Typ-I-Antigen(e); und
(c) Anweisungen zum Durchführen des immunodiagnostischen Tests.

28. Immunodiagnostisches Test-Kit zum Unterscheiden zwischen *H.pylori-*Typ-Iund *H.pylori*-Typ-II-Infektion in einer biologischen Probe, wobei das Test-Kit umfasst:
(a) ein *H.pylori*-Lysat, umfassend ein oder mehrere *H.pylori*-Typ-gemeinsames/e Antigen(e), immobilisiert auf einem Nitrocellulosestreifen;
(b) ein oder mehrere gereinigte Typ-spezifisches/e *H.pylori*-Typ-I-Antigen(e), immobilisiert auf einem Nitrocellulosestreifen; und
(c) Anweisungen zum Durchführen des immunodiagnostischen Tests.

29. Immunodiagnostisches Test-Kit zur Überwachung eines Patienten, der eine Therapie gegen eine *Helicobacter-pylori*-Infektion vornimmt, wobei das Test-Kit umfasst:
(a) ein *H.pylori*-Lysat, umfassend ein oder mehrere *H.pylori*-Typ-gemeinsames/e Antigen(e), immobilisiert auf einem Nitrocellulosestreifen;
(b) ein oder mehrere gereinigte Typ-spezifisches/e *H.pylori*-Typ-I-Antigen(e), immobilisiert auf einem Nitrocellulosestreifen; und
(c) Anweisungen zum Durchführen des immunodiagnostischen Tests.

30. Immunodiagnostisches Test-Kit nach Anspruch 27, Anspruch 28 oder Anspruch 29, wobei das/die eine oder mehreren Typ-spezifische(n) Antigen(e) aus der Gruppe, bestehend aus einem *H.pylori*-Typ-I-VacA-Polypeptid und einem *H.pylori*-Typ-I-CagA-Polypeptid, ausgewählt ist/sind.

31. Immunodiagnostisches Test-Kit nach Anspruch 27, wobei das/die eine oder mehrere Typ-gemeinsame(n) Antigen(e) in einem *H.pylori-*Lysat bereitgestellt ist/sind.

32. Immunodiagnostisches Test-Kit nach Anspruch 27, wobei das/die eine oder mehrere Typ-gemeinsame(n) Antigen(e) eine *H.pylori*-Urease umfasst /umfassen.

33. Immunodiagnostisches Test-Kit nach Anspruch 28 oder Anspruch 29, wobei das Lysat und das eine oder mehrere Typ-spezifische/e Antigen(e) auf dem gleichen Nitrocellulosestreifen immobilisiert sind.

34. Immunodiagnostisches Test-Kit nach Anspruch 28 oder Anspruch 29, wobei das Lysat und das eine oder mehrere Typ-spezifische/e Antigen(e) auf verschiedenen Nitrocellulosestreifen immobilisiert sind.

## Revendications

1. Procédé de détection d'une infection par Helicobacter pylori chez un sujet ; comprenant :
(a) fournir un échantillon biologique du sujet; et
(b) faire réagir ledit échantillon biologique avec au moins un antigène H. pylori de type courant, et faire réagir l'échantillon avec au moins un antigène purifié spécifique à H. pylori de type I, dans des conditions qui permettent aux anticorps dirigés contre H. pylori, quand ils sont présents dans l'échantillon biologique, de se fixer aux antigènes de type courant, et/ou à (aux) antigène(s) spécifique(s),
détectant, de cette façon, la présence ou l'absence de l'infection par H. pylori, chez le sujet.

2. Procédé selon la revendication 1, dans lequel ladite infection par *H. pylori* est une infection par *H. pylori* de type I.

3. Procédé selon la revendication 1, dans lequel ladite infection par *H. pylori* est une infection par *H. pylori* de type II.

4. Procédé selon l'une quelconque des revendications précédentes, comprenant :
(a) retirer les anticorps non-fixés ;
(b) fournir au moins un fragment capable de s'associer avec lesdits anticorps fixés ; et
(c) détecter la présence ou l'absence dudit au moins un fragment,
détectant, de cette façon, la présence ou l'absence d'une infection par *H. pylori.*

5. Procédé selon la revendication 4, dans lequel ledit au moins un fragment comprend un anticorps anti-immunoglobuline humaine marqué de façon à être décelable.

6. Procédé selon la revendication 5, dans lequel le marqueur décelable est un élément fluorescent ou une enzyme.

7. Procédé selon l'une quelconque des revendications précédentes, dans lequel au moins un desdits antigènes de type courant et au moins un desdits antigènes spécifiques sont immobilisés sur un support solide.

8. Procédé selon la revendication 7, dans lequel ledit au moins un antigène de type courant, et ledit au moins un antigène spécifique sont immobilisés sur le même support solide.

9. Procédé selon la revendication 7, dans lequel ledit au moins un antigène de type courant, et ledit au moins un antigène spécifique sont immobilisés sur des supports solides différents.

10. Procédé selon la revendication 7, la revendication 8 ou la revendication 9, dans lequel le support solide est une bande de nitrocellulose.

11. Procédé pour distinguer entre une infection par *H. pylori* de type I et une infection par *H. pylori* de type II dans un échantillon biologique, ledit procédé comprenant :
(a) immobiliser un lysat de *H. pylori* comprenant au moins un antigène *H. pylori* de type courant sur une bande de nitrocellulose, et immobiliser au moins un antigène purifié et spécifique à *H. pylori* de type I sur une bande de nitrocellulose ;
(b) mettre en contact ladite bande de nitrocellulose de l'étape (a) avec l'échantillon biologique dans des conditions qui permettent aux anticorps anti-*H. pylori* de type I et aux anticorps anti-*H. pylori* de type II, quand ils sont présents dans l'échantillon biologique, de se fixer avec les antigènes *H. pylori* de type courant présents dans ledit lysat et/ou les antigènes spécifiques à *H. pylori* de type I ;
(c) retirer les anticorps non-fixés ;
(d) fournir un anticorps anti-immunoglobuline humaine marqué de façon à être décelable ; et
(e) détecter la présence ou l'absence des anticorps anti-immunoglobuline humaine fixés dans ledit échantillon biologique, détectant, de cette façon, la présence ou l'absence d'une infection par *H. pylori* de type I et/ou une infection par *H. pylori* de type II.

12. Procédé de surveillance d'un sujet subissant une thérapie pour traiter une infection par *H. pylori* comprenant :
(a) fournir un échantillon biologique du sujet ;
(b) immobiliser un lysat de *H. pylori* comprenant au moins un antigène *H. pylori* de type courant sur une bande de nitrocellulose, et immobiliser au moins un antigène purifié et spécifique à *H. pylori* de type I sur une bande de nitrocellulose ;
(c) mettre en contact ladite bande de nitrocellulose de l'étape (b) avec ledit échantillon biologique dans des conditions qui permettent aux anticorps anti-*H. pylori* de type I et aux anticorps anti-*H*. *pylori* de type II, quand ils sont présents dans l'échantillon biologique, de se fixer avec les antigènes *H. pylori* de type courant présents dans ledit lysat et/ou les antigènes spécifiques à *H. pylori* de type I ;
(d) retirer les anticorps non-fixés ;
(e) fournir un anticorps anti-immunoglobuline humaine marqué de façon à être décelable ; et
(f) détecter la présence ou l'absence des anticorps anti-immunoglobuline humaine fixés dans ledit échantillon biologique,
en détectant, de cette façon, le déroulement du traitement de l'infection.

13. Procédé selon la revendication 11 du la revendication 12, dans lequel ledit au moins un antigène de type courant, et ledit au moins un antigène spécifique sont immobilisés sur la même bande en nitrocellulose.

14. Procédé selon la revendication 11 ou la revendication 12, dans lequel ledit au moins un antigène de type courant, et ledit au moins un antigène spécifique sont immobilisés sur des bandes en nitrocellulose différentes.

15. Procédé selon l'une quelconque des revendications précédentes, dans lequel ledit au moins un antigène spécifique est choisi dans la catégorie constituée d'un polypeptide VacA de *H. pylori* de type I et un polypeptide CagA de H. pylori de type I.

16. Procédé selon l'une quelconque des revendications précédentes, dans lequel ledit au moins un antigène de type courant est fourni par un lysat de H. pylori.

17. Procédé selon l'une quelconque des revendications précédentes, dans lequel ledit au moins un antigène de type courant comprend une uréase de *H. pylori.*

18. Procédé selon l'une quelconque des revendications précédentes, dans lequel ledit échantillon biologique est un échantillon de sérum humain.

19. Support de membrane comprenant au moins un antigène *H. pylori* de type courant et au moins un antigène purifié et spécifique à *H. pylori* de type I, immobilisés de façon discrète dessus.

20. Support de membrane selon la revendication 19, dans lequel ledit au moins un antigène spécifique est choisi dans la catégorie constituée d'un polypeptide VacA de *H. pylori* de type I et un polypeptide CagA de H. pylori de type I.

21. Support de membrane selon la revendication 19, dans lequel ledit au moins un antigène de type courant est fourni par un lysat de H. pylori.

22. Support de membrane selon la revendication 19, dans lequel ledit au moins un antigène de type courant comprend une uréase de *H. pylori.*

23. Support de membrane selon l'une quelconque des revendications 19 à 22, comprenant en outre une immunoglobuline humaine, immobilisée de façon discrète dessus.

24. Support de membrane selon l'une quelconque des revendications 19 à 22, dans lequel le support de membrane est une bande de nitrocellulose.

25. Support en nitrocellulose comprenant :
(a) un polypeptide VacA de *H. pylori* de type I ;
(b) un polypeptide CagA de *H. pylori* de type I ;
(c) une uréase de *H. pylori ;* et
(d) une IgG humaine,
dans lequel lesdits polypeptides de *H. pylori* et l'uréase, et ladite IgG humaine sont immobilisés sous forme de bandes discrètes sur ledit support en nitrocellulose.

26. Support en nitrocellulose comprenant :
(a) un polypeptide VacA de *H. pylori* de type I ;
(b) un polypeptide CagA de *H. pylori* de type I ;
(c) un lysat de *H. pylori ;* et
(d) une IgG humaine,
dans lequel lesdits polypeptides de *H. pylori* et le lysat, et ladite IgG humaine, sont immobilisés sous forme de bandes discrètes sur ledit support en nitrocellulose.

27. Kit de test immunodiagnostique pour détecter une infection par *H. pylori,* ledit kit de test comprenant :
(a) au moins un antigène *H. pylori* de type courant ;
(b) au moins un antigène purifié spécifique à *H. pylori* de type I ; et
(c) les instructions pour mener le test immunodiagnostique.

28. Kit de test immunodiagnostique pour faire la distinction entre une infection par H. pylori de type I et une infection par H. pylori de type II dans un échantillon biologique, ledit kit de test comprenant :
(a) un lysat de *H. pylori* comprenant au moins un antigène *H. pylori* de type courant immobilisé sur une bande en nitrocellulose ;
(b) au moins un antigène purifié spécifique à *H. pylori* de type I immobilisé sur une bande en nitrocellulose ; et
(c) les instructions pour mener le test immunodiagnostique.

29. Kit de test immunodiagnostique pour surveiller un sujet ,subissant une thérapie pour une infection par Helicobacter pylori, ledit kit de test comprenant :
(a) un lysat de *H. pylori* comprenant au moins un antigène *H. pylori* de type courant immobilisé sur une bande en nitrocellulose ;
(b) au moins un antigène purifié spécifique à *H*. *pylori* de type I immobilisé sur une bande en nitrocellulose ; et
(c) les instructions pour mener le test immunodiagnostique.

30. Kit de test immunodiagnostique selon la revendication 27, la revendication 28 ou la revendication 29, dans lequel ledit au moins un antigène spécifique est choisi dans la catégorie constituée d'un polypeptide VacA de *H. pylori* de type I et un polypeptide CagA de H. pylori de type I.

31. Kit de test immunodiagnostique selon la revendication 27, dans lequel ledit au moins un antigène de type courant est fourni par un lysat de H. pylori.

32. Kit de test immunodiagnostique selon la revendication 27, dans lequel ledit au moins un antigène de type courant comprend une uréase de *H. pylori.*

33. Kit de test immunodiagnostique selon la revendication 28 ou la revendication 29, dans lequel ledit lysat et ledit au moins un antigène spécifique sont immobilisés sur la même bande en nitrocellulose.

34. Kit de test immunodiagnostique selon la revendication 28 ou la revendication 29, dans lequel ledit lysat et ledit au moins un antigène spécifique sont immobilisés sur des bandes en nitrocellulose différentes.
